# EUROPÄISCHE PATENTANMELDUNG

(11) **EP 1 982 745 A2**
(43) Veröffentlichungstag der Anmeldung: **22.10.2008**
(21) Anmeldenummer: 08006202.9
(22) Anmeldetag: 29.03.2008
(51) Int. Cl.: A61N 1/372

(54) **Eine Follow-Up Untersuchung unterstützende Therapieeinheit sowie Therapiesystem**

(30) Priorität: 21.04.2007 DE 102007018982
(71) Anmelder: BIOTRONIK CRM Patent AG, 6341 Baar (CH)
(72) Erfinder: Reinke, Joachim, 10439 Berlin (DE); Bungartz, Jörn, 13086 Berlin (DE); Kukla, Volker, Dr., 12157 Berlin (DE); Rau, Tobias, 10779 Berlin (DE)
(74) Vertreter: Lindner-Vogt, Karin L.

(57) **Zusammenfassung**

Die Erfindung betrifft Therapiesystem mit einer implantierbaren (Sensor- und) Therapieeinheit, die eine Steuereinheit, einen mit der Steuereinheit verbundenen Speicher, eine wenigstens indirekt mit der Steuereinheit verbundene Telemetrieeinheit zu drahtlosen bidirektionalen Übertragung von Daten von und zu einem externen Gerät, sowie eine Erfassungseinheit zum Erfassen von physiologischen Daten eines Patienten oder von Betriebsdaten des implantierbaren medizinischen Gerätes aufweist, wobei die Steuereinheit ausgebildet ist, eine ausgehende Datenübertragung von der implantierbaren Therapieeinheit zum externen Gerät aufgrund vorgegebener Therapiegerät-interner Ereignisse auszulösen und eine Empfangsbereitschaft der Telemetrieeinheit zum Empfangen des Beginns (Headers) einer eingehenden Datenübertragung vom externen Gerät zur Therapieeinheit ausschließlich innerhalb eines vorgegebenen Antwort-Zeitfensters nach einer Datenübertragung von der implantierbaren Therapieeinheit zum externen Gerät herzustellen, wobei das System ausgebildet ist einer eingehenden Datenübertragung Follow-Up Signalisierungsdaten hinzuzufügen, die eine bevorstehende Follow-Up Untersuchung signalisieren, wobei die Steuereinheit weiterhin ausgebildet ist, in Reaktion auf den Empfang von Follow-Up Signalisierungsdaten die Sensoreinheit zu einem vorgegebenen Zeitpunkt dazu zu veranlassen, vorgegebene, für eine Follow-Up Untersuchung benötigte physiologische Daten oder Betriebsdaten der Therapieeinheit zu erfassen und in dem Speicher zu speichern, und diese Daten mit einer nachfolgenden ausgehenden Datenübertragung zu dem externen Gerät zu übertragen.

## Beschreibung

Die Erfindung betrifft ein Therapiesystem mit einer implantierbaren Therapieeinheit, beispielsweise einem implantierbaren medizinischen Gerät wie einem Herzschrittmacher oder einem Kardioverter/Defibrillator.

Derartige implantierbare medizinische Geräte als Therapieeinheiten können mit einer bidirektionalen Telemetrieeinheit ausgestattet sein, um auf diese Weise über ein patientennahes externes Gerät (Patientengerät) mit einem zentralen, entfernten Service-Center kommunizieren zu können. Ein den jeweiligen Patienten betreuender Arzt hat patientenspezifischen Zugang zu dem Service-Center und kann von dem Service-Center beispielsweise physiologische oder implantatsbezogene Daten abrufen, die zuvor von der Therapieeinheit über das Patientengerät zu dem Service-Center übertragen worden.

Da die drahtlose Datenübertragung vom implantierbaren medizinischen Gerät zum Patientengerät auch auf Seiten des implantierbaren medizinischen Gerätes aufzuwendende Energie braucht, die in einem implantierbaren medizinischen Gerät durch eine Batterie nur begrenzt zur Verfügung gestellt wird, ist die bidirektionale Datenkommunikation zwischen dem implantierbaren medizinischen Gerät und dem Patientengerät üblicherweise beschränkt. Beispielsweise ist es vorgesehen, dass das implantierbare medizinische Gerät nur dann eine ausgehende Datenübertragung auslöst, wenn dies beispielsweise zu bestimmten Zeiten vorgesehen ist oder wenn dies durch ein besonderes, seitens des implantierbaren medizinischen Gerätes detektierten Ereignisses wie beispielsweise einer akuten Fibrillation angezeigt ist.

Um Energie zu sparen, ist es vorgesehen, dass das implantierbare medizinische Gerät nicht ständig empfangsbereit ist. Vielmehr hält das implantierbare medizinische Gerät nur für eine kurze Zeit nach Abschluss einer ausgehenden Datenübertragung für ein AntwortZeitfenster die Empfangsbereitschaft aufrecht. Alternativ oder zusätzlich kann eine Empfangsbereitschaft auch innerhalb eines jeweiligen Empfangsfensters zu in der Therapieeinheit voreingestellten Zeiten bestehen. Wenn innerhalb des Antwort-Zeitfensters oder des Empfangs-Zeitfensters keine von dem Patientengerät zum implantierbaren medizinischen Gerät gerichtete eingehende Datenübertragung erfolgt, schaltet das implantierbare medizinische Gerät seine Empfangsbereitschaft wieder ab. Auf diese Weise ist es möglich, dass eine ausgehende Datenübertragung vom implantierbaren medizinischen Gerät (also der Therapieeinheit) zum Patientengerät innerhalb des vorgegebenen Antwort-Zeitfensters (Empfangszeitfensters) mit einer den Empfang der ausgehenden Datenübertragung quittierenden eingehenden Datenübertragung beantwortet wird. Dabei ist es möglich, der eingehenden Datenübertragung außer dem reinen Quittierungssignal weitere Daten hinzuzufügen.

Aufgabe der Erfindung ist es, eine Follow-Up Untersuchung (Nachsorgeuntersuchung) für einen Patienten mit einem implantierbaren medizinischen Gerät, beispielsweise einem Herzschrittmacher, möglichst effizient zu gestalten. Anlässlich einer Follow-Up Untersuchung wertet ein untersuchender Arzt eine Reihe von seitens des implantierbaren medizinischen Gerätes erfasster Daten aus, wie beispielsweise Daten zu aktuellen Reizschwellen für effektive Stimulationsimpulse bei einem Herzschrittmacher, Daten zu einer von dem implantierbaren medizinischen Gerät erfassten natürlichen (intrinsischen) Herzrate eines Patienten, Daten, die anzeigen, wie viel Spannung notwendig ist, um das Herz zu einer Kontraktion zu stimulieren, Daten zur Impulsbeschaffenheit, Daten zum Ladezustand der Batterie, Daten zur Funktionsfähigkeit von an das implantierbare medizinische Gerät angeschlossenen Elektrodenleitungen und weitere.

Erfindungsgemäß wird die Aufgabe durch ein Therapiesystem gelöst, welches eine Therapieeinheit, also insbesondere ein implantierbares medizinisches Gerät, wie einen Herzschrittmacher oder einen Defibrillator aufweist. Die Therapieeinheit umfasst eine Steuereinheit und einen mit der Steuereinheit verbundenen Speicher sowie eine wenigstens indirekt mit der Steuereinheit verbundene Telemetrieeinheit zur drahtlosen bidirektionalen Übertragung von Daten von und zu einem externen Gerät, beispielsweise zu einem Patientengerät oder auch direkt zu einem Service-Center. Außerdem umfasst die Therapieeinheit eine Erfassungseinheit zum Erfassen physiologischer Daten eines Patienten, also beispielsweise der intrinsischen Herzrate, der aktuellen Reizschwelle für die Elektrostimulation, Ergebnisse eines retrograden Leitungstestes sowie von Betriebsdaten der Therapieeinheit, die beispielsweise eine aktuelle Stimulationsimpulsbeschaffenheit, die Funktionsfähigkeit von an die Therapieeinheit angeschlossenen Elektrodenleitungen oder den Zustand der Batterie der Therapieeinheit widerspiegeln.

Die Steuereinheit der Therapieeinheit ist ausgebildet, eine ausgehende Datenübertragung von der implantierbaren Therapieeinheit zu einem externen Gerät aufgrund vorgegebener Therapiegerät-interner Ereignisse auslösen, also beispielsweise zu Therapiegerät intern festgelegten Zeitpunkten oder beim Detektieren bestimmter, vorgegebener Ereignisse. Die Steuereinheit hält eine Empfangsbereitschaft der Telemetrieeinheit zum Empfangen des Beginns (Headers) einer eingehenden Datenübertragung vom externen Gerät zur Therapieeinheit ausschließlich innerhalb eines vorgegebenen Antwort-Zeitfensters nach einer ausgehenden Datenübertragung von der implantierbaren Therapieeinheit zum externen Gerät oder innerhalb zeitlich vorgegebener Empfangszeitfenster aufrecht.

An dieser Stelle ist anzumerken, dass für die Zwecke dieser Beschreibung die Begriffe "eingehende Datenübertragung" und "ausgehende Datenübertragung" mit Bezug auf die implantierbare Therapieeinheit verwendet werden. Eine ausgehende Datenübertragung im Sinne dieser Beschreibung ist somit eine, die bei einem externen Gerät oder auch beim Service-Center eingeht.

Das Therapiesystem ist ausgebildet, einer eingehenden, innerhalb des Antwort-Zeitfensters oder des Empfangs-Zeitfensters abzusendenden Datenübertragung Follow-Up-Signalisierungsdaten hinzuzufügen, die eine bevorstehende Follow-Up Untersuchung signalisieren. Die Steuereinheit der implantierbaren Therapieeinheit ist ausgebildet, in Reaktion auf den Empfang einer eingehenden Datenübertragung, die Follow-Up-Signalisierungsdaten enthält, die Sensoreinheit zu einem vorgegebenen Zeitpunkt dazu zu veranlassen, vorgegebene, für eine Follow-Up Untersuchung benötigte physiologische Daten oder Betriebsdaten der Therapieeinheit zu erfassen und in dem Speicher der implantierbaren Therapieeinheit zu speichern. Außerdem veranlasst die Steuereinheit der implantierbaren Therapieeinheit die Übertragung dieser so gesammelten Daten für eine Follow-Up Untersuchung mit einer nachfolgenden ausgehenden Datenübertragung zu dem externen Gerät.

Ein derartiges Therapiesystem bietet mehrere Vorteile. Zum einen können seitens des implantierbaren Therapiegerätes anzustellende Voruntersuchungen zu einer Follow-Up Untersuchung zeitnah vor einer Follow-Up Untersuchung durchgeführt werden, so dass die erfassten Daten aktuell sind, aber während der eigentlichen Follow-Up Untersuchung nicht mehr ermittelt werden müssen. Dies spart wertvolle Zeit während der eigentlichen Follow-Up Untersuchung. Darüber hinaus hat die so initiierte automatische Ermittlung der für die Follow-Up Untersuchung erforderlichen Daten den Vorteil, dass notwendige Untersuchungen nicht vergessen werden, wie das ansonsten der Fall sein könnte, wenn ein Arzt diese Untersuchungen wie bisher üblich anlässlich einer Follow-Up Untersuchung Schritt für Schritt manuell (beispielsweise mittels eines Programmiergerätes) auslösen muss.

Gemäß einer ersten Ausführungsvariante des Therapiesystems ist die Steuereinheit der implantierbaren Therapieeinheit so ausgebildet, dass der vorgegebene Zeitpunkt, zu dem die Therapieeinheit das Erfassen der Daten für die Follow-Up Untersuchung veranlasst, unmittelbar nach dem Empfang der Follow-Up Signalisierungsdaten liegt.

Bei diesem System umfasst das Therapiesystem vorzugsweise ein Service-Center, das für die bidirektionale Datenkommunikation mit der implantierbaren Therapieeinheit (gegebenenfalls über ein externes Gerät) ausgebildet ist und das das Absenden an die implantierbare Therapieeinheit gerichteten eingehenden Datenübertragungen mit Follow-Up Signalisierungsdaten zu einem Voruntersuchungszeitpunkt auslöst, an dem die Voruntersuchungen für eine bevorstehende Follow-Up Untersuchung begonnen werden sollen. Dazu weist das Service-Center in der bevorzugten Ausführungsvariante einen Datenspeicher zum Speichern des Datums einer bevorstehenden Follow-Up Untersuchung auf und ist ausgebildet, ausgehend von dem im Datumsspeicher gespeicherten Datum einen Voruntersuchungszeitpunkt zu berechnen, der eine vorgegebene Zeit vor dem im Datumsspeicher gespeicherten Datum für eine kommende Follow-Up Untersuchung liegt. Das Service-Center ist weiter dazu ausgebildet, dass es die eingehende, Follow-Up Signalisierungsdaten enthaltende Nachricht als Antwort auf eine von der Therapieeinheit ausgehende Nachricht veranlasst, die seitens des Service-Centers oder des Patientengerätes nach dem zuvor ermittelten Voruntersuchungszeitpunkt empfangen wird. Bei dieser Ausführungsvariante erfolgt die Berechnung des Voruntersuchungszeitpunktes somit im Service-Center.

Alternativ kann das Therapiesystem auch so ausgebildet sein, dass die implantierbare Therapieeinheit einen mit der Steuereinheit verbundenen Zeit- und Datumsgeber aufweist und die Steuereinheit dazu ausgebildet ist, in Reaktion auf den Empfang von Follow-Up Signalisierungsdaten aus diesen Follow-Up Signalisierungsdaten ein Datum einer kommenden Follow-Up Untersuchung zu extrahieren, um dann den eine vorgegebene Zeit vor dem Datum für die kommenden Follow-Up Untersuchung liegenden Voruntersuchungszeitpunkt zu bestimmen. Dieser so von der implantierbaren Therapieeinheit bestimmter Voruntersuchungszeitpunkt wird in dem Speicher der implantierbaren Therapieeinheit gespeichert. Die Steuereinheit ist weiter dazu ausgebildet, die Sensoreinheit zu dem gespeicherten Voruntersuchungszeitpunkt dazu zu veranlassen, die für die Follow-Up Untersuchung benötigten Daten zu erfassen. Weiterhin veranlasst die Steuereinheit anschließend die Übertragung dieser Daten mit einer nachfolgenden ausgehenden Datenübertragung.

Bei dieser Ausführungsvariante wird somit der eigentliche Voruntersuchungszeitpunkt in der implantierbaren Therapieeinheit selbst bestimmt. Dementsprechend ist ein Service-Center als Bestandteil des Therapiesystems vorzugsweise so ausgebildet, dass es das Datum einer bevorstehenden Follow-Up Untersuchung sobald wie möglich an die implantierbare Therapieeinheit überträgt, also nicht erst nachdem der vorgesehene Voruntersuchungszeitpunkt verstrichen ist, sondern vielmehr schon dann, wenn der nächste Follow-Up Untersuchungszeitpunkt feststeht.

Zum Bestimmen eines Datums für eine Follow-Up Untersuchung ist vorzugsweise eine Eingabeeinheit vorgesehen, die direkt oder indirekt mit dem Service-Center verbunden ist und das Eingeben eines Datums einer kommenden Follow-Up Untersuchung erlaubt. Die Eingabe eines derartigen Datums hat dann das Hinzufügen von Follow-Up Signalisierungsdaten zu einer eingehenden Datenübertragung entweder nach dem zu bestimmenden Voruntersuchungszeitpunkt oder kurzfristig nach Eingabe des Datums für die Follow-Up Untersuchung zur Folge.

Im Hinblick darauf, dass das Sammeln der für eine Follow-Up Untersuchung erforderlichen Daten seitens der implantierbaren Therapieeinheit genau wie die Datenübertragung selbst einen Teil der nur begrenzt zur Verfügung stehenden Energie erfordert, ist es vorteilhaft, wenn entweder die implantierbare Therapieeinheit so ausgebildet ist, dass sie das Sammeln von Daten im Rahmen einer Voruntersuchung zur Follow-Up Untersuchung nur nach vorgegebenen Mindestzeitabständen zu einer vorangegangenen Voruntersuchung zulässt oder das Service-Center so ausgebildet ist, dass es Follow-Up Signalisierungsdaten enthaltende eingehende Datenübertragung nur in bestimmten Mindestzeitabständen zulässt. Damit kann vermieden werden, dass zu viele Follow-Up Voruntersuchungen ausgelöst werden und damit unnötig viel Energie verbraucht wird.

Neben dem Therapiesystem als Ganzem ist auch eine einzelne implantierbare Therapieeinheit als Lösung der vorgenannten Aufgabe vorgesehen. Diese Therapieeinheit unterscheidet sich von an sich bekannten Therapieeinheiten zumindest dadurch, dass ihre Steuereinheit dazu ausgebildet ist, in Reaktion auf den Empfang einer eingehenden Datenübertragung, die Follow-Up Signalisierungsdaten enthält, die Sensoreinheit zu einem vorgegebenen Zeitpunkt dazu zu veranlassen, vorgegebene, für eine Follow-Up Untersuchung benötigte physiologische Daten oder Betriebsdaten der Therapieeinheit zu erfassen und in dem Speicher der implantierbaren Therapieeinheit zu speichern. Im Weiteren ist die Steuereinheit dazu ausgebildet, die Übertragung dieser Daten mit einer ausgehenden Datenübertragung nach Abschluss des Sammelns der Daten zu veranlassen. Das besondere Merkmal einer derartigen implantierbaren Therapieeinheit besteht somit darin, dass sie aufgrund der Ausgestaltung ihrer Steuereinheit dazu in der Lage ist, Signalisierungsdaten in eine eingehende Datenübertragung zu erkennen, geeignet auszuwerten und daraufhin das Sammeln von physiologischen Daten und/oder Betriebsdaten der implantierbaren Therapieeinheit zu veranlassen.

Gemäß einer weiteren vorteilhaften Variante der Therapieeinheit ist diese ausgebildet, das Sammeln von für eine Follow-Up Untersuchung benötigten Daten zu dem durch die Follow-Up Signalisierungsdaten bestimmten Zeitpunkt dann zu unterlassen, wenn zu diesem Zeitpunkt die Batterie der Therapieeinheit unter ein vorgegebenes Grenzmaß hinaus entleert ist.

Eine vorteilhafte alternative Variante des Therapiesystems besteht darin, dass die Therapieeinheit ggf. infolge einer eingehenden Datenübertragung, die dementsprechende Follow-Up Signalisierungsdaten enthält, so eingestellt ist, dass die Therapieeinheit in vorgegebenen Zeitabständen, die vorzugsweise einige Monate betragen, von sich aus das Sammeln von für eine Follow-Up Untersuchung benötigten Daten auslöst.

Weitere vorteilhafte Ausgestaltungen einer derartigen implantierbaren Therapieeinheit ergeben sich aus den vorstehenden Ausführungen zu dem Therapiesystem.

Die Erfindung soll nun anhand eines Ausführungsbeispiels mit Bezug auf die Figuren näher erläutert werden. Von den Figuren zeigen
- Figur 1:: ein Therapiesystem mit einer implantierbaren Therapieeinheit, einem externen Gerät (Patientengerät) und einem Service-Center;
- Figur 2:: eine detailliertere Darstellung der implantierbaren Therapieeinheit in Form eines Zweikammer-Herzschrittmachers in Verbindung mit dem Patientengerät,

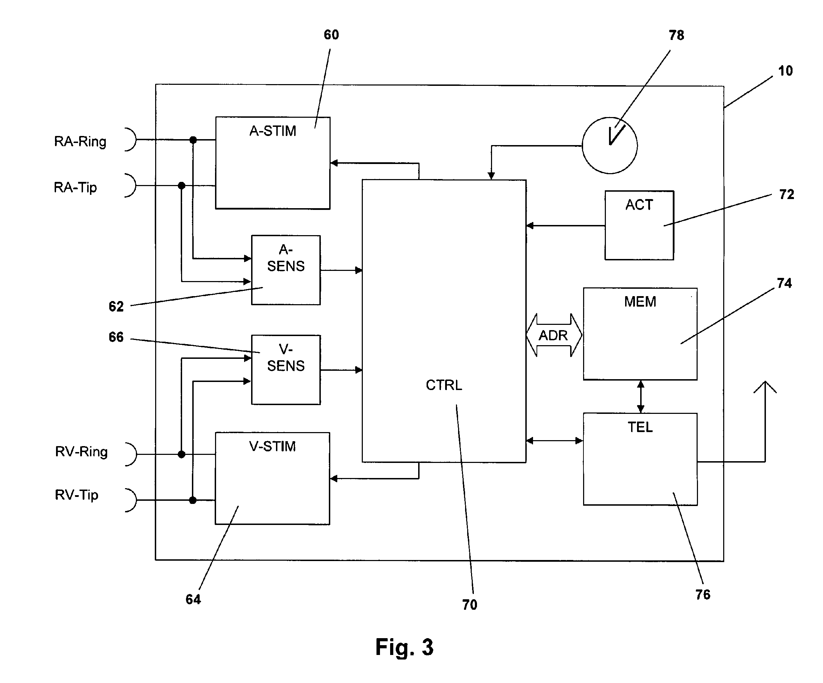

- Figur 3:: ein Blockschaltbild der implantierbaren Therapieeinheit.

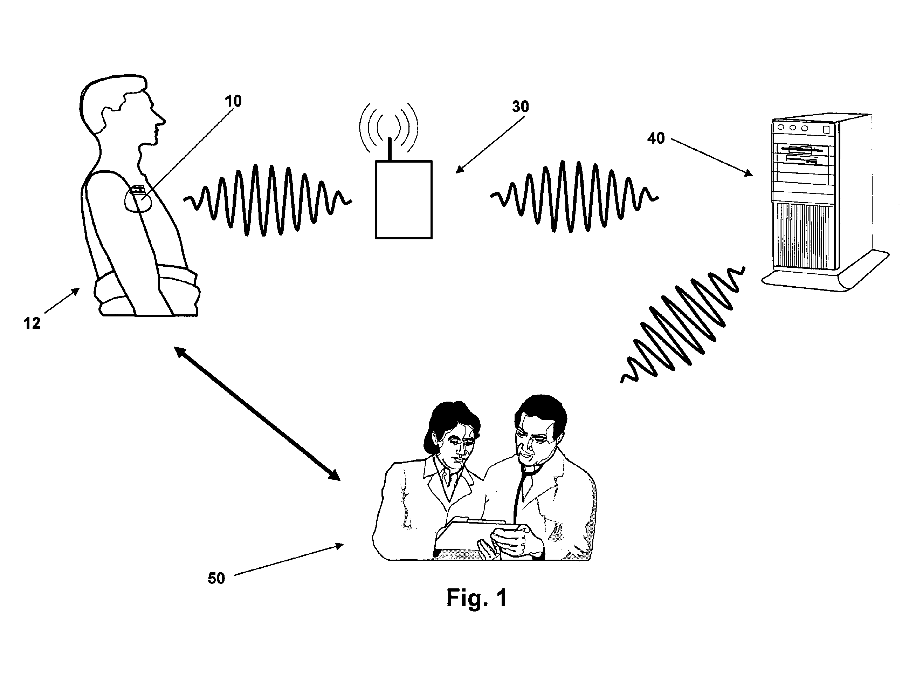

Zu dem in Fig. 1 abgebildeten Therapiersystem zählen eine implantierbare Therapieeinheit 10, die in diesem Falle ein in einem Patienten 12 implantierter Herzschrittmacher ist. Weitere Systembestandteile sind ein externes Gerät 30 sowie ein Service-Center 40.

Das externe Gerät 30 - auch Patientengerät genannt - sowie die implantierte Therapieeinheit 10 sind dazu ausgebildet, mit vergleichsweise kurzer Reichweite bidirektional drahtlos Daten auszutauschen. Daher verbleibt das externe Gerät 30 in der Regel in der Nähe des Patienten 12.

Außerdem ist das externe Gerät 30 dazu ausgebildet, eine bidirektionale Datenverbindung über eine größere Entfernung mit einem zentralen Service-Center 40 herzustellen, welches in Fig. 1 durch einen Server repräsentiert ist.

Ein Arzt 50 (oder ein Ärzteteam) hat die Möglichkeit, in dem Service-Center 40 zu einer spezifischen implantierbaren Therapieeinheit abgelegte Daten abzufragen. Außerdem ist das Service-Center 40 so ausgebildet, dass es einem Arzt 50 beispielsweise mittels SMS oder e-mail regelmäßig Nachrichten zu einer jeweiligen individuellen implantierbaren Therapieeinheit 10 oder zum Gesundheitszustand des entsprechenden Patienten 12 übermittelt. Umgekehrt hat der Arzt 50 die Möglichkeit, in dem Service-Center 40 Daten zu hinterlegen, beispielsweise Daten, die das Datum einer festgesetzten, zukünftigen Nachsorgeuntersuchung (Follow-Up Untersuchung) für den Patienten 12 betreffen.

Im Rahmen einer solchen Nachsorgeuntersuchung tritt der Arzt 50 in direkten Kontakt mit dem Patienten 12, wie durch den zweiseitigen Pfeil in Fig. 1 dargestellt ist. Der Arzt 50 hat dabei die Möglichkeit, mittels eines nicht dargestellten Programmiergerätes unmittelbar bidirektional mit der implantierten Therapieeinheit 10 zu kommunizieren. Nach heutigem Stand der Technik werden für eine Nachsorgeuntersuchung benötigte Daten vom Arzt selbst während einer Nachsorgeuntersuchung mittels eines Programmiergerätes und der implantierbaren Therapieeinheit 10 beschafft.

Wie eingehend bereits erläutert, ist die implantierbare Therapieeinheit 10 so ausgebildet, dass sie bei vorgegebenem Anlass oder zu einem vorgegebenen Zeitpunkt von sich aus eine ausgehende Datenübertragung zu dem externen Gerät 30 veranlasst und nach Abschluss dieser Datenübertragung nur für eine vorherbestimmte Zeit (ein Antwortzeitfenster) empfangsbereit ist, um den Beginn (zumindest den Header) einer eingehenden, von dem externen Gerät 30 an die implantierte Therapieeinheit 10 gerichteten Datenübertragung zu empfangen. Im einfachsten Fall besteht diese eingehende Datenübertragung ausschließlich aus einer Quittung, die der implantierten Therapieeinheit 10 signalisiert, dass die ausgehende Datenübertragung erfolgreich war, so dass die implantierte Therapieeinheit 10 nicht eine erneute ausgehende Datenübertragung mit denselben Daten veranlassen muss.

Die bidirektionale Datenverbindung zwischen dem externen Gerät 30 und dem Service-Center 40 kann zeitlich unbeschränkt sein und kann sowohl drahtlos wie auch drahtgebunden sein, da auf der Übertragungsstrecke zwischen externem Gerät 30 und Service-Center 40 keine derartige Ressourcenknappheit besteht, wie in Bezug auf die implantierbare Therapieeinheit 10.

Seitens der implantierbaren Therapieeinheit 10 empfangene Daten überträgt das externe Gerät 30 daher in der Regel unverzüglich zum Service-Center 40. Das Service-Center 40 hat die Möglichkeit, über das externe Gerät 30 eine bei der implantierbaren Therapieeinheit 10 eingehende Datenübertragung zu veranlassen, die über ein Quittungssignal hinaus weitergehende Daten enthalten kann.

Die sind im speziellen Ausführungsbeispiel insbesondere Follow-Up Signalisierungsdaten, die die implantierbare Therapieeinheit 10 aufgrund dessen konkreter Ausgestaltung dazu veranlassen, entweder sofort oder zu einem berechneten Voruntersuchungszeitpunkt mit dem Sammeln solcher physiologischer und/oder Betriebs-Daten zu beginnen, die für eine Follow-Up Untersuchung benötigt werden.

Damit erlaubt das Figur 1 dargestellte Therapiesystem das folgende Szenario:

Das Service-Center bietet dem Arzt die Möglichkeit, dass dieser beim Service-Center das Datum einer geplanten Follow-Up Untersuchung hinterlegt.

Zu einem gewünschten Zeitpunkt (je nach Szenario mit der nächsten eingehenden Datenübertragung, die auf die Eingabe eines Follow-Up Untersuchungsdatums hin erfolgt oder erst nach einem zuvor berechneten Voruntersuchungszeitpunkt) veranlasst das Service-Center das externe Gerät 30 zum Absenden einer eingehenden Datenübertragung, die Follow-Up Signalisierungsdaten enthält, welche so beschaffen sind, dass sie die implantierte Therapieeinheit entweder sofort oder zu einem von der implantierbaren Therapieeinheit selbst zu berechnenden Voruntersuchungszeitpunkt zum Sammeln solcher Daten veranlasst, die für eine Follow-Up Untersuchung benötigt werden. Die Veranlassung des externen Gerätes durch das Service-Center ist hier so zu verstehen, dass das Service-Center 40 eine entsprechende Daten enthaltende Datenübertragung zum externen Gerät 30 zu einem jeweils gegebenen Zeitpunkt, also beispielsweise zum Vorsorgeuntersuchungszeitpunkt oder unmittelbar nach Festlegung des Datums für eine Follow-Up Untersuchung auslöst. Dadurch wird das Patientengerät 30 dazu veranlasst, einer nächstmöglichen eingehenden Datenübertragung an die implantierbare Therapieeinheit 10 die entsprechenden Follow-Up Signalisierungsdaten hinzuzufügen.

Diese eingehende Datenübertragung erfolgt, sobald das externe Gerät 30 eine ausgehende Datenübertragung seitens der implantierbaren Therapieeinheit 10 empfangen hat.

Nach Empfang der entsprechenden Follow-Up Signalisierungsdaten enthaltenden eingehenden Datenübertragung veranlasst die implantierbare Therapieeinheit 10 - genauer gesagt: deren Steuereinheit - das Sammeln der entsprechenden Daten, indem es beispielsweise ein Messen der Reizschwelle auslöst, im Messen der intrinsischen Herzrate für einen retrograden Leitungstest durchführt, die Impulsbeschaffenheit übermittelt, einen Elektrodencheck durchführt oder Ähnliches.

Die somit ermittelten Daten überträgt die implantierbare Therapieeinheit 10 mit der nächsten anfallenden ausgehenden Datenübertragung an das externe Gerät 30, welches diese Daten weiter zum Service-Center 40 überträgt, wo diese Daten dann dem Arzt 50 während einer dann durchzuführenden Follow-Up Untersuchung zur Verfügung stehen.

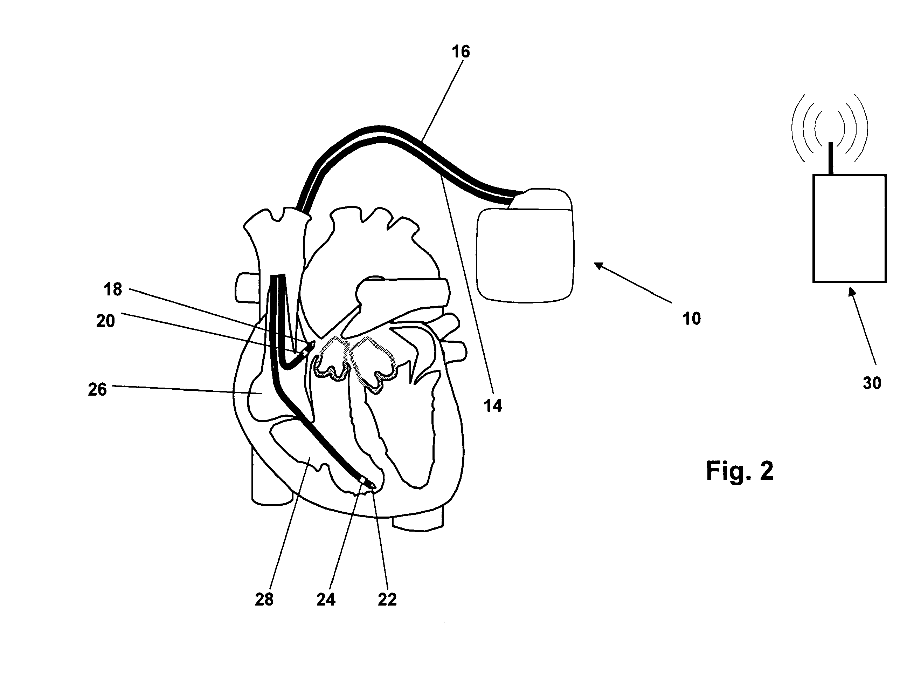

Die implantierbare Therapieeinheit ist vorzugsweise ein implantierbares medizinisches Gerät wie ein Herzschrittmacher oder ein Kardioverter/Defibrillator. Figur 2 zeigt einen Zweikammer-Herzschrittmacher als implantierbare Therapieeinheit 10. Dieser Zweikammer-Herzschrittmacher ist über Elektrodenleitungen 14 und 16 mit Stimulations- und Sensingelektroden 18 und 20 beziehungsweise 22 und 24 im Atrium beziehungsweise Ventrikel eines Herzens verbunden und kann auf diese Weise Stimulationsimpulse an das Herz abgeben und elektrische Potentiale vom Herzen aufnehmen.

Dazu sind die Elektrodenleitungen 14 und 16 entsprechend mit einer atrialen Stimulationseinheit 60, einer atrialen Sensingeinheit 62, einer ventrikulären Stimulationseinheit 64 und einer ventrikulären Stimulationseinheit 66 verbunden. Diese Einheiten sind wiederum mit einer zentralen Steuereinheit 70 des Herzschrittmachers 10 verbunden. Die zentrale Steuereinheit 70 des Herzschrittmachers 10 ist mittels der Sensingeinheiten 62 und 66 dazu in der Lage, eine intrinsische atriale oder ventrikuläre Herzate eines Patienten zu erfassen oder auch die Wirksamkeit eines über einen der Stimulationseinheiten 60 oder 64 abgegebenen atrialen oder ventrikulären Stimulationsimpulses, um so auf an sich bekannte Weise eine atriale oder ventrikuläre Reizschwelle zu ermitteln. Darüber hinaus ist die Steuereinheit 70 mit einem Aktivitätssensor 72 verbunden, um eine jeweilige Stimulationsrate an den hämodynamischen Bedarf eines Patienten anpassen zu können.

Weiterhin ist die Steuereinheit 70 mit einem Speicher 74 verbunden, der zum einen Steuerbefehle für die Steuereinheit 70 enthalten kann, aber auch als Speicher für seitens der Steuereinheit 70 mittels der Stimulationseinheiten 60 und 64 sowie der Sensingeinheiten 62 und 66 ermittelte physiologische Daten eines Patienten dient. Außerdem dient der Speicher 74 als Pufferspeicher für eine Datenübertragung, für die der Speicher 74 mit einer Telemetrieeinheit 76 verbunden ist. Die Telemetrieeinheit 76 kann auch direkt und ausschließlich mit der Steuereinheit 70 verbunden sein. Die Steuereinheit 70 steuert die Telemetrieeinheit 76 in der zuvor beschriebenen Weise so, dass sie bei Erfassen bestimmter Ereignisse, beispielsweise ventrikulärer Fibrillationen oder zu bestimmten Zeitpunkten eine ausgehende Datenübertragung veranlasst, bei der im Speicher 74 gespeicherte Daten an das externe Gerät 30 übertragen werden. Die Steuereinheit 70 schaltet die Telemetrieeinheit 76 nach Beendigung einer derartigen ausgehenden Datenübertragung für ein kurzes Antwortzeitfenster auf Empfang. Wenn die Telemetrieeinheit 76 innerhalb dieses Antwortzeitfensters den Header einer eingehenden Datenübertragung empfängt, der der Steuereinheit 70 signalisiert, dass die eingehende Datenübertragung den Zweikammer-Herzschrittmacher 10 betrifft, wird die Empfangsbereitschaft für die Dauer dieser eingehenden Datenübertragung aufrecht erhalten und der Empfang anschließend abgeschaltet. Auf diese Weise können einer eingehenden Datenübertragung zumindest theoretisch beliebig viele Daten hinzugefügt werden.

Im hier beschriebenen, besonderen Ausführungsbeispiel, ist die Steuereinheit 70 dazu ausgebildet, auf in einer eingehenden Datenübertragung enthaltene Follow-Up Signalisierungsdaten anzusprechen und zwar entweder so, dass die Steuereinheit 70 sofort das Sammeln von für eine Follow-Up Untersuchung benötigten Daten veranlasst oder alternativ, indem die Steuereinheit 70 aus den Signalisierungsdaten das Datum einer bevorstehenden Follow-Up Untersuchung extrahiert. Mit Hilfe dieses Datums berechnet die Steuereinheit 70 dann einen geeigneten, vor dem Datum der Follow-Up Untersuchung liegenden Voruntersuchungszeitpunkt, zu dem die Voruntersuchungen für eine Follow-Up Untersuchung begonnen werden müssen. Mit Hilfe eines Zeit- und Datumsgebers 78 löst dann die Steuereinheit 70 das Sammeln der für die Follow-Up Untersuchung benötigten Daten zu dem zuvor ermittelten Voruntersuchungszeitpunkt aus.

Dabei ist die Steuereinheit 70 in dem hier beschriebenen, bevorzugten Ausführungsbeispiel dazu ausgebildet, eine Wiederholung des Sammelns von Daten für eine Follow-Up Untersuchung nur nach vorgegebenen Mindestzeitabständen zuzulassen, um eine Batterieerschöpfung allein durch das Sammeln der Daten für eine Follow-Up Voruntersuchung auszuschließen.

## Patentansprüche

1. Therapiesystem mit einer implantierbaren Therapieeinheit, die
eine Steuereinheit,
einen mit der Steuereinheit verbundenen Speicher,
eine wenigstens indirekt mit der Steuereinheit verbundene Telemetrieeinheit zu drahtlosen bidirektionalen Übertragung von Daten von und zu einem externen Gerät, sowie
eine Erfassungseinheit zum Erfassen von physiologischen Daten eines Patienten oder von Betriebsdaten des implantierbaren medizinischen Gerätes aufweist,
wobei die Steuereinheit ausgebildet ist, eine ausgehende Datenübertragung von der implantierbaren Therapieeinheit zum externen Gerät aufgrund vorgegebener Therapiegerät-interner Ereignisse auszulösen und eine Empfangsbereitschaft der Telemetrieeinheit zum Empfangen des Beginns (Headers) einer eingehenden Datenübertragung vom externen Gerät zur Therapieeinheit ausschließlich innerhalb eines vorgegebenen Antwort-Zeitfensters nach einer Datenübertragung von der implantierbaren Therapieeinheit zum externen Gerät oder innerhalb eines Empfangszeitfensters zu in der Therapieeinheit voreingestellten Zeiten herzustellen
**dadurch gekennzeichnet, dass**
das System ausgebildet ist einer eingehenden Datenübertragung Follow-Up Signalisierungsdaten hinzuzufügen, die eine bevorstehende Follow-Up Untersuchung signalisieren,
wobei die Steuereinheit weiterhin ausgebildet ist,
in Reaktion auf den Empfang von Follow-Up Signalisierungsdaten die Sensoreinheit zu einem vorgegebenen Zeitpunkt dazu zu veranlassen, vorgegebene, für eine Follow-Up Untersuchung benötigte physiologische Daten oder Betriebsdaten der Therapieeinheit zu erfassen und in dem Speicher zu speichern, und
diese Daten mit einer nachfolgenden ausgehenden Datenübertragung zu dem externen Gerät zu übertragen.

2. Therapiesystem nach Anspruch 1, **dadurch gekennzeichnet, dass** der vorgegebene Zeitpunkt unmittelbar nach dem Empfang der Follow-Up Signalisierungsdaten liegt.

3. Therapiesystem nach Anspruch 2, **dadurch gekennzeichnet, dass** das Therapiesystem ein Service-Center umfasst, welches dazu ausgebildet ist, bidirektional mit einem externen Gerät für die Datenkommunikation mit der Therapieeinheit zu kommunizieren und welches einen Datumsspeicher zum Speicher eines Datums für eine kommende Follow-Up Untersuchung aufweist und dazu ausgebildet ist, einen eine vorgegebene Zeit vor dem Datum für eine kommende Follow-Up Untersuchung Voruntersuchungszeitpunkt zu bestimmen, und
das externe Gerät dazu zu veranlassen, nach Empfang einer von der Therapieeinheit stammenden ausgehenden Nachricht nach dem Voruntersuchungszeitpunkt eine eingehende, vom externen Gerät zur Therapieeinheit gerichtete Nachricht zu generieren, die Follow-Up Signalisierungsdaten enthält und innerhalb des Antwortzeitfensters an die Therapieeinheit zu übertragen.

4. Therapiesystem nach Anspruch 3, **dadurch gekennzeichnet, dass** das Service-Center dazu ausgebildet ist, das externe Gerät nur dann dazu zu veranlassen, nach Empfang einer von der Therapieeinheit stammenden ausgehenden Nachricht nach dem Voruntersuchungszeitpunkt eine eingehende, vom externen Gerät zur Therapieeinheit gerichtete Nachricht zu generieren, die Follow-Up Signalisierungsdaten enthält und innerhalb des Antwortzeitfensters an die Therapieeinheit zu übertragen, wenn seit einer vorangegangenen Übertragung von Signalisierungsdaten an die Therapieeinheit ein vorgegebener Ruhezeitraum verstrichen ist.

5. Therapiesystem nach Anspruch 1, **dadurch gekennzeichnet, dass** die Therapieeinheit einen mit der Steuereinheit verbundenen Zeit- und Datumsgeber aufweist und die Steuereinheit dazu ausgebildet ist,
in Reaktion auf den Empfang von Follow-Up Signalisierungsdaten aus den Follow-Up Signalisierungsdaten ein Datum einer kommenden Follow-Up Untersuchung zu extrahieren und einen eine vorgegebene Zeit vor dem Datum für eine kommende Follow-Up Untersuchung Voruntersuchungszeitpunkt zu bestimmen, und
die Sensoreinheit zu dem Voruntersuchungs-Zeitpunkt als dem vorgegebenen Zeitpunkt dazu zu veranlassen, die vorgegebenen, für eine Follow-Up Untersuchung benötigten physiologischen Daten oder Betriebsdaten der Therapieeinheit zu erfassen und in dem Speicher zu speichern, und
diese Daten mit einer nachfolgenden ausgehenden Datenübertragung zu dem externen Gerät zu übertragen.

6. Therapiesystem nach Anspruch 5, **dadurch gekennzeichnet, dass** das Therapiesystem ein Service-Center umfasst, welches dazu ausgebildet ist, bidirektional mit einem externen Gerät für die Datenkommunikation mit der Therapieeinheit zu kommunizieren und welches einen Datumsspeicher zum Speicher eines Datums für eine kommende Follow-Up Untersuchung aufweist und dazu ausgebildet ist,
das externe Gerät dazu zu veranlassen, nach Empfang einer von der Therapieeinheit stammenden ausgehenden Nachricht eine eingehende, vom externen Gerät zur Therapieeinheit gerichtete Nachricht zu generieren, die die Follow-Up Signalisierungsdaten einschließlich eines Datums einer kommenden Follow-Up Untersuchung enthält und innerhalb des Antwortzeitfensters an die Therapieeinheit zu übertragen.

7. Therapiesystem nach Anspruch 3 oder 6, dass das Service-Center wenigstens indirekt mit einer Eingabeeinheit zum Eingeben des Datums einer kommenden Follow-Up Untersuchung aufweist.

8. Therapiesystem nach einem der Ansprüche 1 bis 7, **dadurch gekennzeichnet, dass** die Therapieeinheit dazu ausgebildet ist, die Sensoreinheit nur dann zu veranlassen, die vorgegebenen, für eine Follow-Up Untersuchung benötigten physiologischen Daten oder Betriebsdaten der Therapieeinheit zu erfassen und in dem Speicher zu speichern, und diese Daten mit einer nachfolgenden ausgehenden Datenübertragung zu dem externen Gerät zu übertragen,
wenn seit einem vorangegangen Erfassen oder Übertragen der Daten mehr als ein vorgegebener Ruhezeitraum verstrichen ist.

9. Therapieeinheit für ein System gemäß eines der Ansprüche 1 bis 8, **dadurch gekennzeichnet, dass** die Therapieeinheit
eine Steuereinheit,
einen mit der Steuereinheit verbundenen Speicher,
eine wenigstens indirekt mit der Steuereinheit verbundene Telemetrieeinheit zu drahtlosen bidirektionalen Übertragung von Daten von und zu einem externen Gerät, sowie
eine Erfassungseinheit zum Erfassen von physiologischen Daten eines Patienten oder von Betriebsdaten des implantierbaren medizinischen Gerätes aufweist,
wobei die Steuereinheit ausgebildet ist, eine ausgehende Datenübertragung von der implantierbaren Therapieeinheit zum externen Gerät aufgrund vorgegebener Therapiegerät-interner Ereignisse auszulösen und eine Empfangsbereitschaft der Telemetrieeinheit zum Empfangen des Beginns (Headers) einer eingehenden Datenübertragung vom externen Gerät zur Therapieeinheit ausschließlich innerhalb eines vorgegebenen Antwort-Zeitfensters nach einer Datenübertragung von der implantierbaren Therapieeinheit zum externen Gerät herzustellen
**dadurch gekennzeichnet, dass**
die Steuereinheit weiterhin ausgebildet ist,
in Reaktion auf den Empfang einer eingehenden Datenübertragung, die Follow-Up Signalisierungsdaten enthält, die Sensoreinheit zu einem vorgegebenen Zeitpunkt dazu zu veranlassen, vorgegebene, für eine Follow-Up Untersuchung benötigte physiologische Daten oder Betriebsdaten der Therapieeinheit zu erfassen und in dem Speicher zu speichern, und
diese Daten mit einer nachfolgenden ausgehenden Datenübertragung zu dem externen Gerät zu übertragen.
